# EUROPEAN PATENT APPLICATION

(11) **EP 4 659 675 A1**
(43) Date of publication of application: **10.12.2025**
(21) Application number: 25180549.5
(22) Date of filing: 03.06.2025
(51) Int. Cl.: A61B 6/00, A61B 6/58

(54) **INFORMATION PROCESSING APPARATUS, MEDICAL IMAGING SYSTEM, IMAGE PROCESSING METHOD, IMAGE PROCESSING PROGRAM, AND X-RAY IMAGING SYSTEM**

(30) Priority: 07.06.2024 JP 2024093103; 05.03.2025 JP 2025034607
(71) Applicant: Canon Medical Systems Corporation, Tochigi 324-0036 (JP)
(72) Inventor: AOYAMA, Gakuto, Otawara-shi, 324-0036 (JP); HIRANO, Yoshinori, Otawara-shi, 324-0036 (JP)
(74) Representative: Marks & Clerk LLP

(57) **Abstract**

According to one embodiment, an information processing apparatus includes processing circuitry. The processing circuitry is configured to acquire a camera image including an examination portion of an object. The processing circuitry is further configured to input a prompt including at least the camera image into a generative model to acquire a virtual medical image from the generative model. The processing circuitry is further configured to set a main imaging condition, which is an imaging condition used for a main imaging, based on the virtual medical image, and to output the main imaging condition to a medical imaging apparatus.

## Description

### FIELD

Embodiments described herein relate generally to an information processing apparatus, a medical imaging system, an image processing method, an image processing program, and an X-ray imaging system.

### BACKGROUND

In diagnosis using medical images, the examination portion of an object to be diagnosed or treated must be properly imaged by a medical imaging apparatus such as an X-ray imaging apparatus, CT (Computed Tomography) apparatus, or other medical imaging apparatus. It is desirable that the appropriate imaging condition is set according to examinations such that clinically desired, i.e., user desired, medical images are acquired. However, depending on the experience of the user such as a technician, the appropriate imaging condition may not be set and the desired medical images may not be acquired.

For example, in simple X-ray imaging in orthopedics, an object may be placed in a specific posture and radiographed at a predetermined angle. In such cases, when the imaging condition is inappropriate, the desired medical image may not be obtained, or the object may be exposed to unnecessary X-ray exposure due to the need for another imaging. Hence, the imaging condition for radiography may be set based on the human body model patterns of the reference body position for each age group of men and women stored in the database.

### SUMMARY OF INVENTION

One of the problems to be solved by the embodiments disclosed in the specification and figures is to set an imaging condition to acquire clinically desirable medical images without increasing radiation exposure. However, the problems to be solved by the embodiments disclosed herein and in figures are not limited to the above problem. Problems corresponding to each effect of each configuration shown in each embodiment described below can also be other problems.

According to one embodiment, an information processing apparatus includes processing circuitry. The processing circuitry is configured to acquire a camera image including an examination portion of an object. The processing circuitry is further configured to input a prompt including at least the camera image into a generative model to acquire a virtual medical image from the generative model. The processing circuitry is further configured to set a main imaging condition, which is an imaging condition used for a main imaging, based on the virtual medical image, and to output the main imaging condition to a medical imaging apparatus.

According to one embodiment, an information processing apparatus includes processing circuitry. The processing circuitry is configured to input a camera image including an examination portion of an object and a prompt into a generative model to acquire a virtual medical image from the generative model. The processing circuitry is further configured to set a main imaging condition, which is an imaging condition used for a main imaging, based on the virtual medical image, and to output the main imaging condition to a medical imaging apparatus.

The virtual medical image may be a medical image generated based on a provisional imaging condition.

The virtual medical image may be a medical image imaged at a position and angle of an X-ray tube from which the camera image was acquired.

The virtual medical image may be a simulation of a medical image from a medical imaging apparatus. The medical imaging apparatus being selected from X-ray or MRI.

The processing circuitry may be configured to set the main imaging condition based on a geometric positional relationship between an imaging portion of the object and an X-ray tube used in the generation of the virtual medical image.

The processing circuitry may be configured to: acquire a provisional imaging condition, and the camera image including the examination portion of the object; input the prompt including the provisional imaging condition and the camera image to the generative model; and acquire the virtual medical image from the generative model.

The imaging condition may include at least one of the following conditions: imaging range, imaging distance, position of an X-ray tube, and angle of the X-ray tube.

The processing circuitry is configured to acquire an object information including information about a posture of the object, and to set the main imaging condition based on the object information and the virtual medical image.

The processing circuitry may be configured to input the prompt including the object information and the virtual medical image to the generative model for imaging conditions, and to set an imaging condition generated by the generative model of imaging conditions as the main imaging conditions.

The processing circuitry may be configured to control a display of the virtual medical image on a display.

The processing circuitry may be configured to acquire an imaging condition input to an input interface, and to reset the main imaging condition based on the imaging condition input to the input interface.

The processing circuitry may be configured to calculate at least one of an imaging range, an imaging distance, a position of an X-ray tube, and an angle of the X-ray tube to acquire a medical image based on a positional relationship between skeletal structure in the virtual medical image, and to set the calculated condition as the main imaging condition.

The processing circuitry may be configured to set the main imaging condition by: acquiring the camera image for one examination portion of the object and input the prompt including the camera image to the generative model to acquire a plurality of virtual images from the generative model; and selecting one imaging condition for acquiring a desired medical image from a plurality of imaging conditions for the respective plurality of virtual medical images.

The processing circuitry may be configured to: acquire a plurality of camera images, wherein there is a camera image for each examination portion for a plurality of examination portions of the object; input the prompt including the plurality of camera images to the generative model to acquire the virtual medical image for each examination portion from the generative model; set the main imaging condition for each examination portion based on the virtual medical image for each examination portion; and control the output of the medical imaging condition for each examination portion.

According to one embodiment, a medical imaging system, including the aforementioned information processing apparatus, a medical imaging apparatus connected to the information processing apparatus via a network and an image sensor connected to the information processing apparatus via the network.

According to one embodiment, an image processing method, comprising: acquiring a camera image including an examination portion of an object; inputting a prompt including at least the camera image into a generative model to acquire a virtual medical image from the generative model; setting a main imaging condition, which is an imaging condition used for a main imaging, based on the virtual medical image; and outputting the main imaging condition to a medical imaging apparatus.

According to one embodiment, an image processing method, comprising: inputting a camera image including an examination portion of an object and a prompt into a generative model to acquire a virtual medical image from the generative model; setting a main imaging condition, which is an imaging condition used for a main imaging, based on the virtual medical image, and outputting the main imaging condition to a medical imaging apparatus.

According to one embodiment, an image processing program for causing a computer to perform the following steps: acquire a camera image including an examination portion of an object; input a prompt including at least the camera image into a generative model to acquire a virtual medical image from the generative model; set a main imaging condition, which is an imaging condition used for a main imaging, based on the virtual medical image; and output the main imaging condition to a medical imaging apparatus.

According to one embodiment, an image processing program for causing a computer to perform each component of the aforementioned information processing apparatus.

According to one embodiment, an X-ray imaging system includes X-ray irradiator irradiates X-rays to an object, X-ray detector detects the X-rays transmitted through the object, and processing circuitry. The processing circuitry is configured to acquire a camera image including an examination portion of the object. The processing circuitry is further configured to input a prompt including at least the camera image into a generative model to acquire a virtual medical image from the generative model. The processing circuitry is further configured to set a main imaging condition, which is an imaging condition used for a main imaging, based on the virtual medical image, and to control a position or angle of at least one of the X-ray irradiator and the X-ray detector based on the main imaging condition.

### BRIEF DESCRIPTION OF THE DRAWINGS

Fig. 1 is a block diagram showing an example configuration of a medical imaging system including an information processing apparatus according to the present embodiment.
Fig. 2 is a block diagram showing an example configuration of the medical imaging apparatus connected to the information processing apparatus according to the present embodiment.
Fig. 3 is a flowchart showing an example of an operation of the information processing apparatus according to the first embodiment.
Fig. 4 is a diagram illustrating an example of the operation of the information processing apparatus according to the first embodiment.
Fig. 5 is a schematic diagram of the skeletal structure of the front and side of the right knee.
Fig. 6A is an example of right knee front imaging in the first imaging condition.
Fig. 6B is an example of a virtual medical image in Fig. 6A.
Fig. 7A is an example of right knee front imaging in the second imaging condition.
Fig. 7B is an example of a virtual medical image in Fig. 7A.
Fig. 8A is an example of right knee lateral imaging in the first imaging condition.
Fig. 8B is an example of a virtual medical image in Fig. 8A.
Fig. 9A is an example of right knee lateral imaging in the second imaging condition.
Fig. 9B is an example of virtual medical image in Fig. 9A.
Fig. 10A is another example of right knee front imaging in the first imaging condition.
Fig. 10B is an example of virtual medical image in Fig. 10A.
Fig. 11A is another example of right knee front imaging in the second imaging condition.
Fig. 11B is an example of virtual medical image in Fig. 11A.
Fig. 12 is a diagram explaining imaging conditions for abdominal frontal imaging.
Fig. 13A is a diagram explaining a placement of an object.
Fig. 13B is a diagram explaining settings of virtual medical images and imaging conditions for multiple examination portions.
Fig. 14 is a flowchart showing an example of an operation of the information processing apparatus according to the second embodiment.
Fig. 15 is a diagram illustrating an example of the operation of the information processing apparatus according to the second embodiment.
Fig. 16A is a diagram explaining right knee lateral imaging.
Fig. 16B is an example of a virtual medical image in a predetermined posture desired in the examination.
Fig. 16C is an example of a virtual medical image in a case where the knee is excessively internally rotated.
Fig. 16D is an example of a virtual medical image in a case where the knee is excessively externally rotated.
Fig. 17A is a diagram explaining right knee front imaging.
Fig. 17B is an example of a virtual medical image of right knee lateral imaging.
Fig. 17C is an example of a virtual medical image when the angle of the tube is not set to the desired angle for the skyline method.
Fig. 17D is an example of a virtual medical image when the angle of the tube is set to the desired angle for the skyline method.
Fig. 18 is a flowchart showing an example of an operation of the information processing apparatus according to a variant of the second embodiment.
Fig. 19 is a diagram illustrating an example of the operation of the information processing apparatus according to the variant of the second embodiment.

### DETAILED DESCRIPTION

Hereinbelow, a description will be given of an information processing apparatus, a medical imaging system, an image processing method, an image processing program, and an X-ray imaging system according to embodiments of the present invention with reference to the drawings. In each figure, identical elements are marked with the same numerals and omitted from the explanation.

### (Medical imaging system)

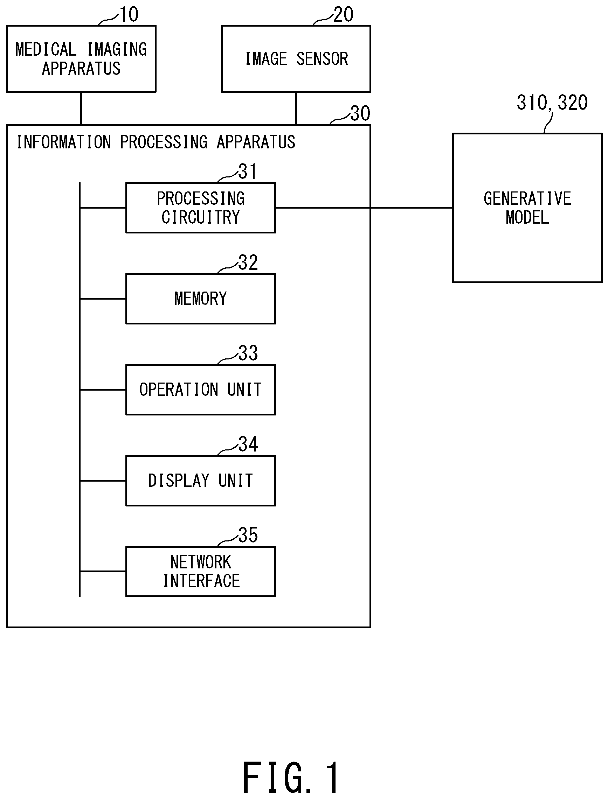

Fig. 1 is a block diagram showing an example configuration of a medical imaging system 1 including an information processing apparatus 30 according to the present embodiment. The medical imaging system 1 includes a medical imaging apparatus 10, an image sensor 20, and the information processing apparatus 30.

### (information processing apparatus)

The information processing apparatus 30 is based on a computer and includes processing circuitry 31, memory 32, operation unit 33, display unit 34, and network interface 35.

The processing circuitry 31 is a processor that reads and executes an image processing program stored in the memory 32 or directly embedded in the processing circuitry 31 to realize each of the functions F01-F09 described below. The processing circuitry 31 also controls each component of the medical imaging apparatus 10. The processing circuitry 31 may also control each component of the image sensor 20.

The memory 32 comprises a recording medium readable by the processor, such as Random Access Memory (RAM), a semiconductor memory element, a hard disk, an optical disk, etc. The memory 32 may be configured, for example, by portable media such as a Universal Serial Bus (USB) memory and Digital Versatile Disk (DVD). The memory 32 stores the image processing program used in the processing circuitry 31 and data necessary to execute the program. The image processing program may be configured to be downloaded from a network via network interface 35.

The operation unit 33 includes an input device and input circuitry. The input device may be an input interface and realized, for example, by a trackball, switch, mouse, keyboard, touchpad, touchscreen that integrates the display screen and touchpad, non-contact input device using an optical sensor, or voice input device. When an input device is operated by the user, the input circuitry generates an instruction signal in response to the operation and outputs it to the processing circuitry 31.

The display unit 34 is a display output device such as a liquid crystal display panel, Organic Light Emitting Diode (OLED) display panel, plasma display panel, or organic electro luminescence panel. The display unit 34 displays the camera image acquired by the image sensor 20, information about imaging conditions, and information for adjusting the posture of the object.

The network interface 35 implements various information communication protocols according to the form of the network and performs communication control according to various protocols. The network interface 35 enables wired or wireless communication with the medical imaging apparatus 10, image sensor 20, and generative models 310 and 320 connected via the network, and exchanges information and data.

### (medical imaging apparatus)

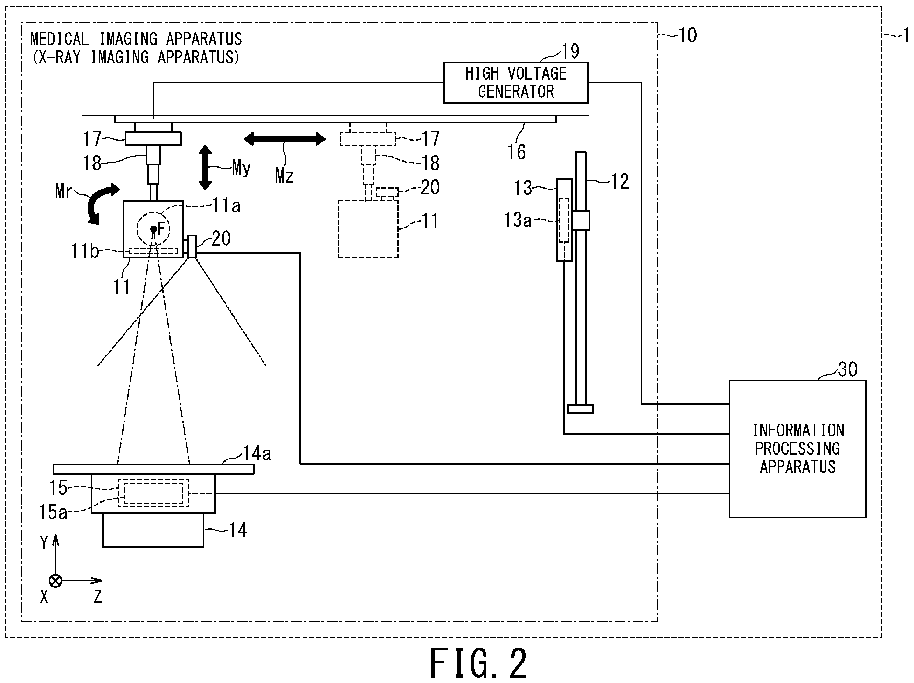

The medical imaging apparatus 10 includes an X-ray imaging apparatus, a CT apparatus, and a magnetic resonance imaging (MRI) apparatus. The X-ray imaging apparatus includes, for example, an X-ray imaging apparatus, an X-ray apparatus for round, an X-ray angiography apparatus, and an X-ray TV apparatus. Fig. 2 is a block diagram showing an example configuration of the medical imaging apparatus 10 connected to the information processing apparatus 30 according to the present embodiment. The case in which the medical imaging apparatus 10 is an X-ray imaging apparatus is described below, but the medical imaging apparatus 10 is not limited to such case.

The X-ray imaging apparatus includes an X-ray irradiation unit 11, a standing examination table 12, a standing position detector unit 13, a bed 14, a lying position detector unit 15, a ceiling rail 16, a movement frame 17, a column 18, and a high voltage generator 19. For the purpose of explanation, the left-right direction of the object placed on the bed 14 is defined as the X-axis direction, the front-back direction (body thickness direction) as the Y-axis direction, and the head-feet direction as the Z-axis direction.

The X-ray irradiation unit (X-ray irradiator) 11 includes an X-ray tube 11a and an X-ray movable aperture 11b. The X-ray tube 11a is referred to as tube. The high voltage generator 19 generates a high voltage to be applied between the anode and cathode and outputs it to X-ray tube 11a. High-voltage power is supplied from the high voltage generator 19 to the X-ray tube 11a, and the X-ray tube 11a irradiates X-rays to the examination portion of the object in front of the standing examination table 12 or on the bed 14.

The X-ray movable aperture 11b comprises a plurality of aperture blades. The aperture blades are composed of, for example, flat lead blades to shield the X-rays. The area surrounded by the multiple aperture blades forms an aperture through which X-rays pass. The X-ray irradiation range formed by the X-ray movable aperture 11b is hereinafter referred to as the "imaging range". The shape, size, and position of the X-ray irradiation range changes according to the conditions related to the "imaging range".

The X-ray irradiation unit 11 is engaged to the column 18 such that the X-ray irradiation unit 11 can be rotated in the direction Mr around an axis (i.e., X axis) that is perpendicular to the extension/retraction direction of the column 18 and passes through the X-ray focus F of the X-ray tube 11a. The X-ray tube 11a is rotated around the X axis (or Y axis, Z axis) passing through the X-ray focus F in the range of -180 to +180 degrees. The angle of rotation of the X-ray tube 11a is hereinafter referred to as the "angle of the tube". The angle of incidence of X-rays changes according to the conditions related to the "angle of the tube".

The standing position detector unit 13 detects X-rays from the X-ray tube 11a. The standing examination table 12 supports the standing position detector unit 13 opposite to the X-ray irradiation unit 11. The height of the standing position detector unit 13 is changed along the standing examination table 12 according to the change in height of the X-ray irradiation unit 11.

The standing position detector unit 13 includes a standing position FPD (Flat Panel Detector) 13a and an A/D (analog to digital) conversion circuit (not shown). The X-ray detector has a plurality of detector elements arranged in two dimensions and detects X-rays. The X-ray detector detects the X-rays transmitted through the object and outputs them as image signals to the information processing apparatus 30. Based on the image signals, an X-ray image (i.e., a medical image) is generated. The X-ray detector may be moved integrally with the X-ray tube 11a.

The bed 14 is used as a lying position examination table, on which a lying or seated object is placed. The bed 14 is supported on the floor and has a top plate 14a on which the object is placed. The top plate 14a can slide and move in the X-axis and Z-axis directions, move up and down and roll in the Y-axis direction.

The lying position detector unit 15 is supported by the bed 14. The lying position detector unit 15 moves in parallel with the X-axis and Z-axis directions of the X-irradiation unit 11. The lying position detector unit 15 includes a lying position FPD 15a and an A/D conversion circuit (not shown). The lying position FPD 15a is an example of an X-ray detector. The lying position detector unit 15 is substantially the same as the standing position detector unit 13, except that lying position detector is used for the object in the lying or seated position instead of the standing position.

The ceiling rail 16 is laid on the ceiling of the room in which the medical imaging apparatus 10 is installed.

The movement frame 17 supports the x-ray irradiation unit 11 via the column 18. The movement frame 17 is engaged to the ceiling rail 16 such that the X-ray irradiation unit 11 can be moved between the standing examination table 12 and the bed 14 in the Z-axis direction Mz along the ceiling rail 16. The movement frame 17 may be installed to be movable in the X-axis direction Mx in addition to the Z-axis direction Mz along the ceiling rail 16. The movement frame 17 can also change the distance Source Image-receptor Distance (SID) between the X-ray tube 11a (X-ray focus F) and the standing position FPD 13a.

The column 18 is supported by the movement frame 17 and supports the X-ray irradiation unit 11 at its lower end. The column 18 is engaged to the movement frame 17 such that it can be moved in the Y-axis direction My. The column 18 is extendable and retractable along the Y-axis direction My. The column 18 can change the distance SID between the X-ray tube 11a (X-ray focus F) and the lying position FPD 15a.

The distance SID between the X-ray tube 11a (X-ray focus F) and the X-ray detector is hereinafter referred to as the " imaging distance". The X-ray irradiation range is expanded or reduced according to the conditions related to the "imaging distance". The position of the X-ray tube 11a in relation to the object is referred to as the "position of the tube". The X-ray irradiation position with respect to the object moves along the direction perpendicular to the direction of the distance SID between the X-ray tube 11a (X-ray focus F) and the X-ray detector according to the "position of the tube" condition. The imaging condition set for imaging by the medical imaging apparatus 10 includes at least one of the following conditions: imaging range, imaging distance, position of the tube, and angle of the tube. The imaging condition also includes conditions related to the tube voltage, tube current, imaging time, mAs value, and grid.

### (image sensor)

The image sensor 20 acquires a camera image. The camera image is an image taken by various cameras. The camera image includes the examination portion of the object whose X-rays are detected by the X-ray detector. The camera image can be either a still image or a moving image. The image sensor 20 performs optical photography. The image sensor 20 includes, for example, a Charge Coupled Device (CCD) camera, Complementary Metal Oxide Semiconductor (CMOS) cameras, and TV cameras. In addition, the image sensor 20 may be an infrared camera that can determine the size and posture of the object without being disturbed by the inspection clothing.

The image sensor 20 may be attached such that it can track the X-ray irradiation position that moves in conjunction with the movement of the X-ray irradiation unit 11 that holds the X-ray tube 11a, and track the X-ray incidence angle towards the object that changes in conjunction with the rotation around the X-ray focus F of the X-ray tube 11a. The image sensor 20 may be attached, for example, to the vicinity of the output port of the X-ray irradiation unit 11 that holds the X-ray tube 11a, or may be attached to the ceiling or wall of the room where the medical imaging apparatus 10 is installed. At least one image sensor 20 is provided. By photographing the examination portion of the object from different angles using multiple image sensors 20, it is also possible to detect the posture of the object in three dimensions using the principle of a stereo camera.

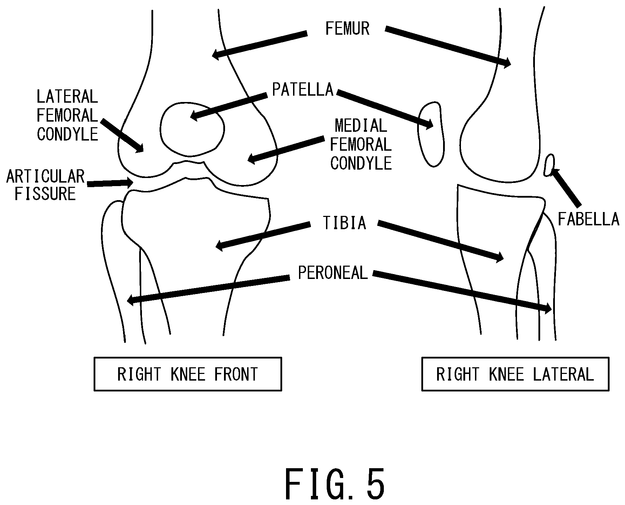

Here, the clinically desirable medical images for knee joints will be described using the schematic diagrams of the right knee joint in Fig. 5 (front and side views). Medical images are X-ray images generated using image signals based on X-rays that have passed through the object. For example, in the case of a frontal view of the knee joint, it is desirable to obtain a medical image that can be read, in which the femur and tibia do not overlap, i.e., the articular fissure J1 located between the femur and tibia is visible. In addition, in the case of lateral knee joint radiography, it is desirable to obtain medical images in which the lateral femoral condyle and medial femoral condyle overlap to a suitable degree, and in which the articular fissure J1 can be read. In addition, in the case of frontal and lateral knee joint radiography, it is desirable to obtain medical images in which the articular fissure J1 is in the center of the image.

In order to obtain such desired medical images, it is necessary to set the appropriate imaging conditions. However, when the user lacks experience, the appropriate imaging conditions may not be set, and the need to re-take the image may arise, causing unnecessary X-ray exposure to the object.

Conventionally, the imaging conditions for X-ray radiography have sometimes been set based on the human body model patterns in the standard body position for men and women of different ages stored in a database. However, there is a risk that an appropriate and accurate imaging condition will not be set for objects that deviate from the standard human body model pattern. Since objects with posture constraints cannot be arranged in the prescribed standard position, there is a risk that the desired medical images will not be obtained with imaging conditions set on the premise of having objects arranged in the prescribed standard position. The information processing apparatus 30 of the present embodiment can set appropriate imaging conditions for acquiring the desired medical images even in such cases.

### (First embodiment)

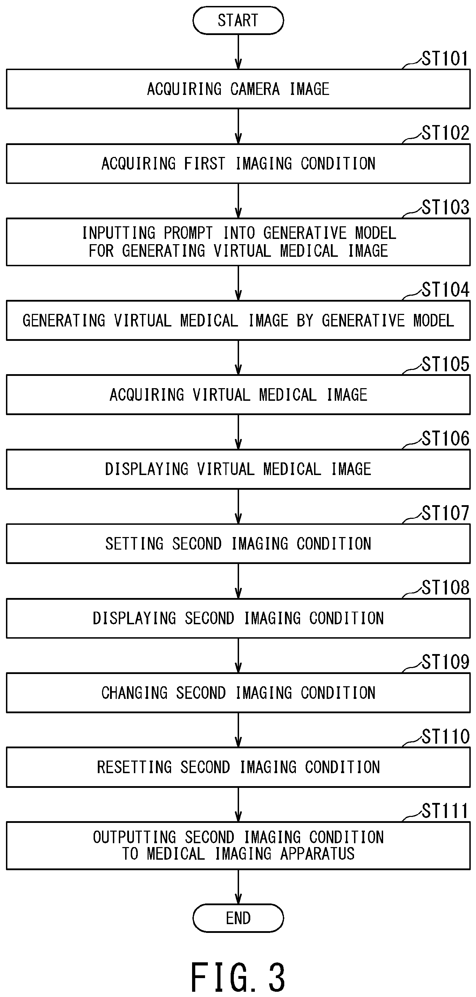

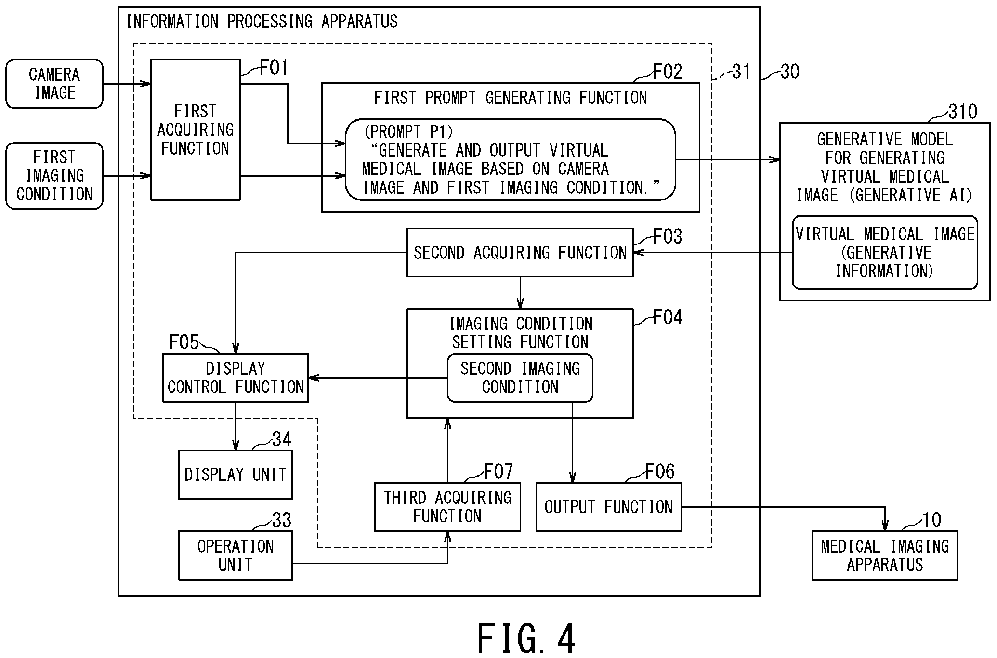

Referring to the flowchart in Fig. 3 and the explanatory diagram in Fig. 4, the functions and operations of the information processing apparatus 30 or the program pertaining to the first embodiment will be explained. Note that the arrows in the explanatory diagrams in Figs. 4, 15, and 19 simply indicate the flow of information.

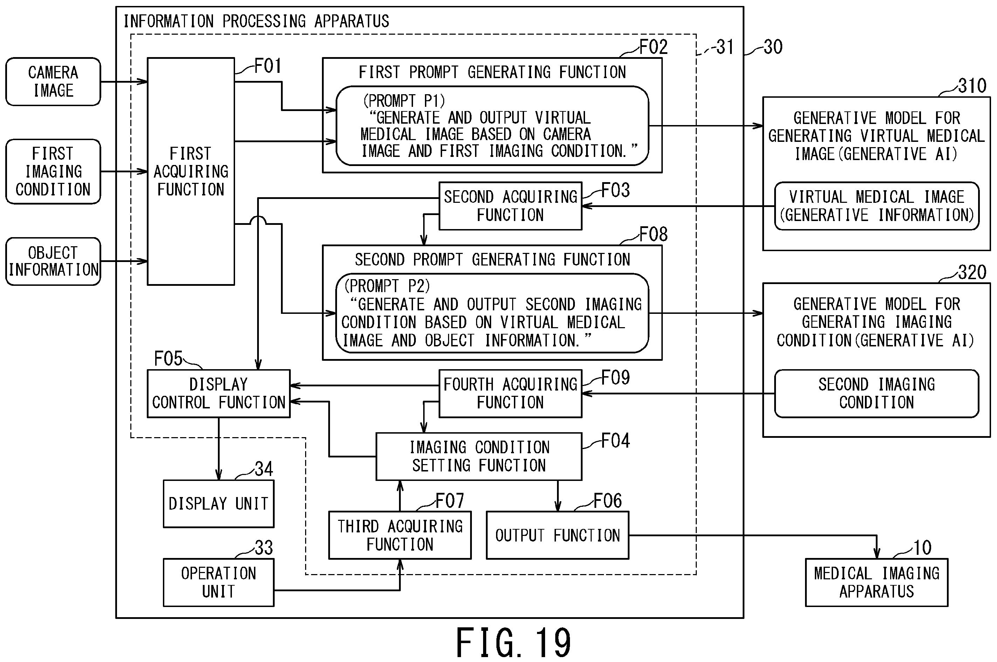

The processing circuitry 31 of the information processing apparatus 30 is a processor that realizes each of the functions of the first acquiring function F01, the first prompt generating function F02, the second acquiring function F03, the imaging condition setting function F04, the display control function F05, the output function F06, the third acquiring function F07, the second prompt generating function F08 (see Fig. 19), and the fourth acquiring function F09 (see Fig. 19).

In step ST101, the first acquiring function F01 acquires a camera image that includes the examination portion of the object. The first acquiring function F01 acquires the camera image using the image sensor 20 connected via a network.

In step ST102, the first acquiring function F01 acquires the first imaging condition. The first imaging condition is a provisional imaging condition, which is a tentative imaging condition. Step ST101 may be carried out after step ST102. In other words, it is also possible to acquire a camera image of the state of the object in the first imaging condition.

The first imaging condition is, for example, the current imaging condition set in the medical imaging apparatus 10 for imaging the examination portion of the object. The first imaging condition may be the imaging condition at the start of the examination by the medical imaging apparatus 10 or a predetermined imaging condition set in accordance with the examination portion.

In step ST103, the first prompt generating function F02 inputs a prompt P1, which includes at least a camera image, to the generative model 310 for the virtual medical image. For example, the first prompt generating function F02 executes a prompt P1 that instructs "Please generate and output a virtual medical image based on the camera image". A virtual medical image is a two-dimensional medical image that is presumed to be imaged under the imaging conditions set for X-ray radiography using the medical imaging apparatus 10. A virtual medical image may be a medical image that is imaged virtually based on provisional imaging conditions. A virtual medical image may be a medical image that is imaged virtually at the position and angle of the tube where the camera image was acquired.

The first prompt generating function F02 may input the prompt P1, which includes the first imaging condition and the camera image, into the generative model 310 for the virtual medical image. In this case, for example, the prompt P1, which instructs "Please generate and output the virtual medical image based on the camera image and the first imaging condition", is executed. In addition, one virtual medical image may be generated for one examination portion, or multiple virtual medical images may be generated. In the above, although the prompt P1 including the camera image is input into the generative model 310 for the virtual medical image here, the camera image may be directly input into the generative model 310. In other words, the prompt P1 and the camera image may be input into the generative model 310 for the virtual medical image.

At step ST104, the generative model 310 for the virtual medical image generates the virtual medical image according to the prompt P1. For example, the network interface 35 exchanges information and data with the generative model 310 for the virtual medical image. Part or all of the generative model 310 for the virtual medical image may be provided in the processing circuitry 31 and the memory 32.

The generative model 310 for the virtual medical image is a generative model constructed using existing medical images as training data, and generates the virtual medical image according to the prompt P1. The generative model 310 for generating the virtual medical image may be a generator of a generative adversarial network (GAN), or it may be a trained model learned using a variational autoencoder (VAE) or a pre-trained model learned using a diffusion model. The generative model 310 for generating the virtual medical image may enhance performance by extracting features and learning latent representations from the camera image, for example. The generative model 310 for the virtual medical image may be constructed to generate virtual medical images using a virtual human body model. For example, an appropriate human body model may be selected based on the camera image. A human body model is, for example, a model that includes at least the human body's skeletal structure, and is a three-dimensional model that is configured such that each body part, such as the head, neck, shoulders, chest, abdomen, upper arm, elbow, forearm, hand, buttocks, thigh, knee, lower leg, and foot, can move.

In step ST105, the second acquiring function F03 acquires the virtual medical image, which is the generated information generated by the generative model 310 for the virtual medical image.

In step ST106, the display control function F05 controls the display of the virtual medical image on the display unit 34. Note that one virtual medical image may be displayed for one examination portion, or multiple virtual medical images may be displayed.

In step ST107, the imaging condition setting function F04 sets the second imaging condition based on the virtual medical image. The second imaging condition is the main imaging condition, which is the imaging condition used for the main imaging. The main imaging is the imaging of the medical image used for medical image diagnosis. When the first imaging condition is determined using the virtual medical image to be appropriate for obtaining the desired medical image, the imaging condition setting function F04 may set the first imaging condition as the second imaging condition.

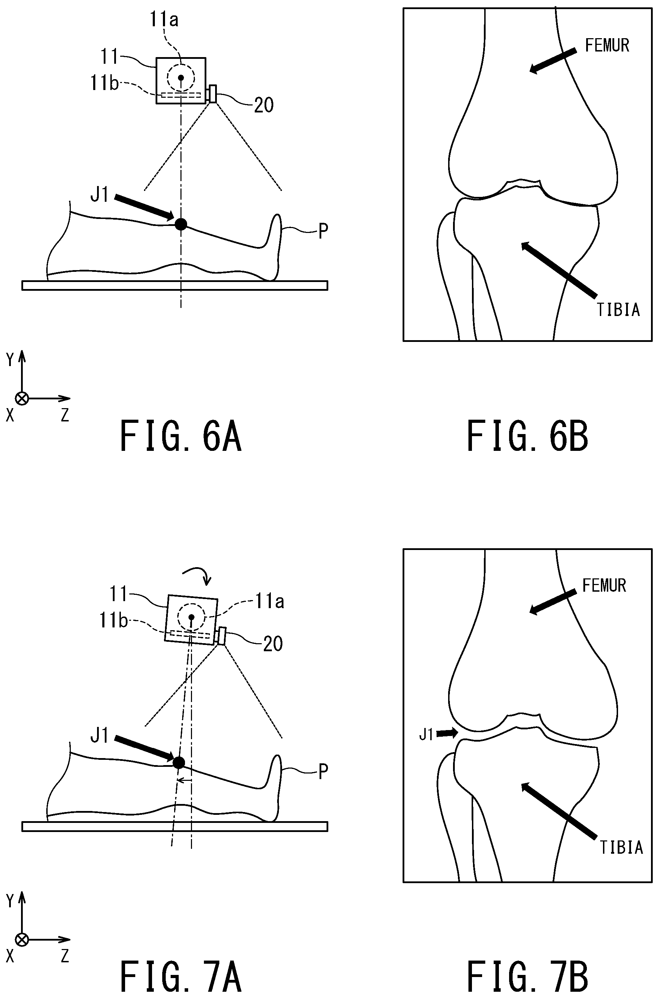

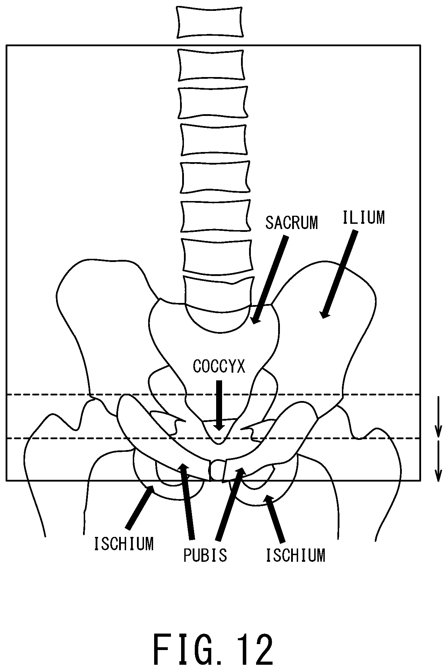

Referring Fig. 6A to Fig. 11B, where the examination portion is the knee, and Fig. 12, where the examination portion is the abdomen, specific examples of how to set the second imaging condition will be described. Fig. 6A shows the right knee front imaging in the first imaging condition. Fig. 6B shows the virtual medical image generated based on the camera image in Fig. 6A. In the virtual medical image in Fig. 6B, the femur and tibia overlap, and the articular fissure J1 cannot be read, therefore as imaging conditions for frontal knee joint imaging, although the imaging range and the position of the tube are appropriate, the angle of the tube is not appropriate.

In this case, the imaging condition setting function F04 calculates the angle of the tube based on the overlapping state of the femur and tibia in the virtual medical image, such that the overlap between the femur and tibia is eliminated and the articular fissure J1 can be read. However, for the case where there is no overlap between the femur and tibia in the virtual medical image and the articular fissure J1 can be read, the imaging condition setting function F04 may determine the second imaging condition without changing the angle of the tube.

Fig. 7A shows a right knee front imaging when the angle of the tube is rotated, for example, by rotating 20 degrees clockwise around the X-axis, so that the overlap between the femur and tibia is eliminated. Fig. 7B is a virtual medical image generated based on the camera image in Fig. 7A. In Fig. 7B, the overlap between the femur and tibia is eliminated and the articular fissure J1 can be read by the angle of the tube calculated based on the positional relationship between the femur and tibia.

The imaging condition setting function F04 can also calculate the angle of the X-ray tube such that the overlap between the femur and tibia is eliminated, based on the geometric positional relationship between the femur and tibia of the object in the virtual medical image generated based on the camera image and the X-ray tube 11a. In other words, the imaging condition setting function F04 sets the main imaging condition based on the geometric positional relationship between the X-ray tube and the imaging portion of the object in the virtual medical image. The area of the imaging portion of the object in the virtual medical image may be wider than the area of the examination portion of the object in the virtual medical image.

The second imaging condition for acquiring the desired medical image may be set based on multiple virtual medical images. In this case, the first acquiring function F01 acquires a camera image for one examination portion of the object. The first prompt generating function F02 inputs a prompt including the camera image into the generative model 310 for the virtual medical image. The second acquiring function F03 acquires multiple virtual medical images from the generative model 310 for the virtual medical image. The imaging condition setting function F04 sets the second imaging condition by selecting one imaging condition for acquiring the desired medical image from the multiple imaging conditions corresponding to the multiple virtual medical images.

When setting the angle of the tube, multiple virtual medical images are acquired for each angle generated by rotating the tube by a predetermined amount, and the angle of the tube of the virtual medical image closest to the desired medical image among the multiple virtual medical images is set as the second imaging condition. For example, among the multiple virtual medical images generated by rotating the angle of the tube from 0 degrees clockwise around the X-axis in increment of 5 degrees, the angle of the tube of the virtual medical image in which the articular fissure J1 is readable is selected and set as the second imaging condition. In this case, the angle of the tube of the virtual medical image in which the area of the articular fissure J1 is the widest may be set, or an interpolated value between angles may be set rather than a predetermined exact value for generating virtual medical images for each angle.

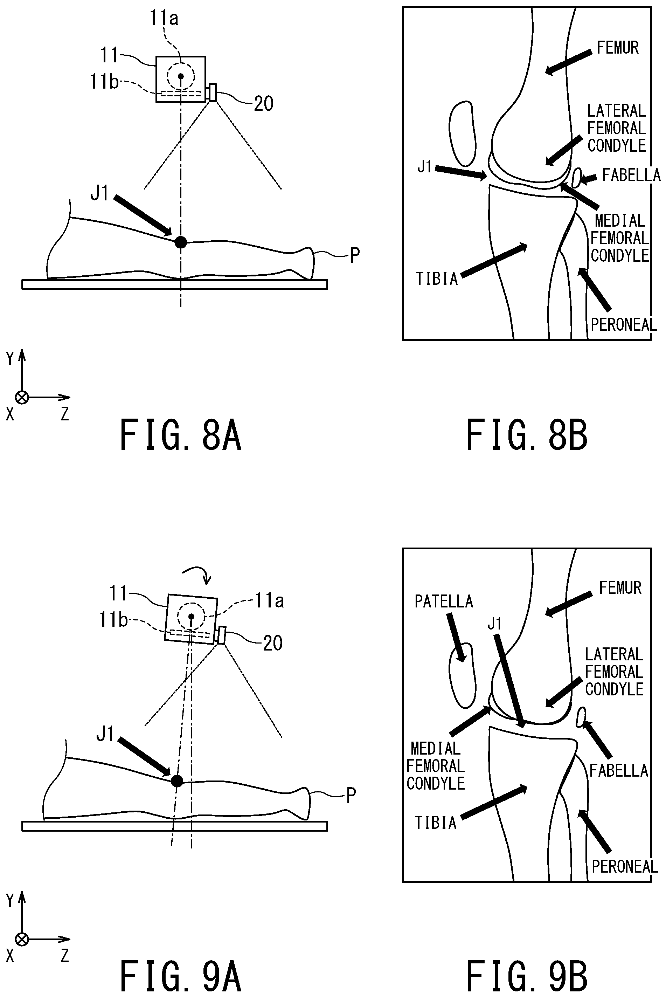

Fig. 8A shows a case of right knee lateral imaging under the first imaging condition. Fig. 8B shows a virtual medical image generated based on the camera image in Fig. 8A. In the virtual medical image in Fig. 8B, the lateral femoral condyle and medial femoral condyle do not overlap, and thus the imaging condition for lateral knee imaging is appropriate in terms of the imaging range and the position of the tube, but the angle of the tube is not appropriate.

In this case, the imaging condition setting function F04 calculates the angle of the tube such that the lateral femoral condyle and medial femoral condyle overlap, based on the state of overlap between the lateral femoral condyle and medial femoral condyle in the virtual medical image. When the lateral femoral condyle and medial femoral condyle overlap in the virtual medical image, the imaging condition setting function F04 may determine the angle of the tube as it is without changing it as the second imaging condition.

Fig. 9A shows a right knee lateral radiograph when the angle of the tube is set such that the lateral femoral condyle and medial femoral condyle overlap, for example, by rotating 10 degrees clockwise around the X-axis. Fig. 9B is a virtual medical image generated based on the camera image in Fig. 9A, and the lateral femoral condyle and medial femoral condyle overlap.

In addition, among the virtual medical images generated by rotating the angle of the tube from 0 degrees clockwise around the X-axis in increment of 5 degrees, the virtual medical image in which the lateral femoral condyle and medial femoral condyle overlap may be selected and set as the second imaging condition. In this case, the angle of the X-ray tube of the virtual medical image may be set so that the overlap between the lateral femoral condyle and medial femoral condyle is the largest, while, in Fig. 9B, the area of the medial femoral condyle that can be read becomes small.

The imaging condition setting function F04 can also calculate the angle of the tube such that the lateral femoral condyle and medial femoral condyle overlap, based on the geometric positional relationship between the X-ray tube 11a and the lateral femoral condyle and medial femoral condyle of the object in the virtual medical image generated based on the camera image. In other words, the imaging condition setting function F04 sets the main imaging condition based on the geometric position relationship between the imaging portion of the object in the virtual medical image and the tube.

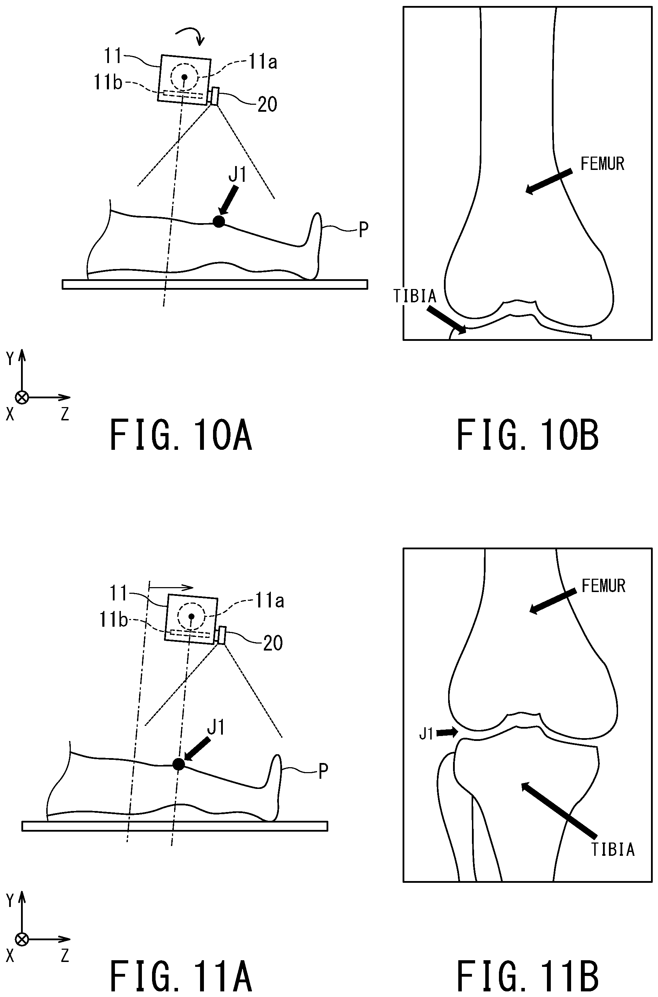

Fig. 10A shows the right knee front imaging in the first imaging condition. Fig. 10B shows the virtual medical image generated based on the camera image in Fig. 10A. In the virtual medical image in Fig. 10B, although the femur and tibia are not overlapping, the knee joint, which is the examination portion of the object, is not located near the center of the virtual medical image. Hence, as the imaging condition for frontal imaging of the knee joint, although the angle of the tube is appropriate, the position of the tube is not appropriate.

In this case, the imaging condition setting function F04 calculates the position of the tube such that the articular fissure J1 is positioned near the center of the virtual medical image, based on the positional relationship between the femur and tibia in the virtual medical image. When the articular fissure J1 is positioned near the center of the virtual medical image, the imaging condition setting function F04 may determine the position of the tube as it is without changing it as the second imaging condition.

Fig. 11A shows a right knee front radiograph when the position of the X-ray tube is set (e.g., by moving the X-ray irradiation unit 11 along the Z-axis), so that the articular fissure J1 is positioned near the center of the virtual medical image. Fig. 11B is a virtual medical image generated based on the camera image in Fig. 11A, and the articular fissure J1 is positioned near the center of the virtual medical image.

The position of the tube for acquiring the desired medical image may be set based on the multiple virtual medical images generated for each amount of movement when the X-ray irradiation unit 11 is moved by a predetermined amount. The imaging condition setting function F04 sets the position of the tube corresponding to the virtual medical image closest to the desired medical image among the multiple virtual medical images for each amount of movement as the second imaging condition. For example, among the multiple virtual medical images generated by moving the X-ray irradiation unit 11 along the Z-axis by 5 cm each, for example, the position of the X-ray tube corresponding to the virtual medical image in which the articular fissure J1 is located near the center of the virtual medical image is selected and set as the second imaging condition.

The imaging condition setting function F04 can also calculate the position of the X-ray tube such that the articular fissure J1 is positioned near the center of the virtual medical image, based on the geometric positional relationship between the X-ray tube 11a and the articular fissure of the object in the virtual medical image generated based on the camera image. In other words, the imaging condition setting function F04 sets the main imaging condition based on the geometric positional relationship between the imaging portion of the object in the virtual medical image and the X-ray tube.

Referring Fig. 12, the imaging conditions for frontal abdominal radiography will be described. In frontal abdominal radiography, when the object can lie down in a position parallel to the top plate 14a, there is little need to adjust the angle of the tube. However, there are times when the clinical field of view is desired to include the pubis in the edge of the medical image, and it is difficult for the user to set such field of view.

Therefore, the imaging condition setting function F04 calculates the conditions for expanding or reducing the imaging range such that the pubis is included in the edge of the virtual medical image. For example, the imaging condition setting function F04 may generate multiple virtual medical images by expanding the imaging range by 3 cm each, for example, and then select the imaging range in which the pubis is included in the edge of the virtual medical image and set it as the second setting condition. The imaging condition setting function F04 may set the position of the tube and the imaging distance such that the pubis is included in the edge of the virtual medical image as the second setting condition.

In this way, the imaging condition setting function F04 calculates at least one of the imaging range, imaging distance, position of the tube, and angle of the tube to acquire the desired medical image based on the positional relationship between the skeletal structure in the virtual medical image, and sets the calculated condition as the second imaging condition.

Returning to Fig. 4, in step ST108, the display control function F05 controls the display unit 34 to display information about the second imaging condition.

In step ST109, the third acquiring function F07 acquires the imaging condition input to the operation unit 33. For example, the user may edit and change the imaging condition from the second imaging condition while referring to the virtual medical image and the information about the second imaging condition set in step ST108 displayed on the display unit 34. By adjusting the imaging condition, the user can set more appropriate imaging conditions. The user may also approve the second imaging condition without changing the imaging condition from the second imaging condition.

In step ST110, the imaging condition setting function F04 resets the second imaging condition based on the imaging condition input to the operation unit 33. The processing in steps ST109 and ST110 is not mandatory, and the second imaging condition set in step ST107 may be output as is to the medical imaging apparatus 10 in step ST111.

In step ST111, the output function F06 controls the medical imaging apparatus 10 connected via the network to output the second imaging condition. The main imaging is performed by the medical imaging apparatus 10 using the second imaging condition.

According to the information processing apparatus 30 of the first embodiment, a virtual medical image is generated based on the camera image, and the second imaging condition used for the main imaging is set based on the virtual medical image. Therefore, an appropriate second imaging condition is set without exposing the object to the amount of radiation associated with pre-imaging, e.g. fluoroscopy for positioning for the main imaging, adjustment of the posture of the object. In addition, because the object is included in the camera image in its current posture, that is, an appropriate second imaging condition is set for the current posture, the object does not need to change to a new posture for the main imaging. Furthermore, because the main imaging is performed under an appropriate second imaging condition, the desired medical image is generated in the main imaging.

### (Variation of the first embodiment)

In the first embodiment, at least one virtual medical image is generated for one examination portion of the object, and one imaging condition is set for the one examination portion. In contrast, the variant of the first embodiment differs from the first embodiment in that at least one virtual medical image is generated and an imaging condition is set for each of the multiple examination portions of the object.

The medical imaging apparatus 10 may be a computed tomography (CT) apparatus. The CT apparatus rotates a pair of an X-ray tube and a detector inside a stand, and irradiates X-rays from multiple directions to an object placed on a bed. The X-ray data that has passed through the object is then analyzed by a computer to generate a three-dimensional cross-sectional image. In CT imaging, a scout image is taken, which is a medical image similar to a general X-ray image, and the second imaging condition is set using the scout image. The scout image is obtained by fixing the tube to a position directly above the top plate or to a specified angle, and then performing CT scan by moving only the top plate within the frame without rotating the frame. The second imaging condition may be determined by using the virtual scout image generated by the generative model 310 for the virtual medical image.

The medical imaging apparatus 10 may be an MRI apparatus. The MRI apparatus executes a scan that excites the atomic nucleus spin of the object placed in a static magnetic field with a radio frequency (RF) pulse of Larmor frequency, and acquires the magnetic resonance (MR) signal generated from the object in conjunction with the excitation, and receives the MR signal acquired by the scan with an RF coil, and generates an MR image based on the MR signal. In imaging using an MRI apparatus, in order to obtain diagnostic images, a positioning image is generated by a locater scan that is performed before the main scan, and the second imaging conditions, such as the imaging range and positioning for the main scan, may be set using the positioning image. The second imaging conditions may be set using the virtual medical image generated by the generative model 310 for the virtual medical image as a virtual positioning image. The second imaging conditions may be set based on the results of the pre-scan performed before the main scan. The results of the pre-scan include, for example, the results of adjustments to static magnetic field inhomogeneity and gain, the imaging portion, the image type, the cross-section, and other adjustments necessary for obtaining good image quality.

In a variant of the first embodiment, the functions and operations or programs shown in Figs. 3 and 4 are executed for each of the multiple examination portions of the object. Specifically, the first acquiring function F01 acquires a camera image for each examination portion of the object. The first prompt generating function F02 inputs a prompt P1, which includes a camera image for each examination portion, into the generative model 310 for the virtual medical image. The prompt P1 may also include a first imaging condition for each examination portion.

The second acquiring function F03 acquires the virtual medical image for each examination portion from the generative model 310 of the virtual medical image. The display control function F05 controls the display of the virtual medical image for each examination portion on the display unit 34.

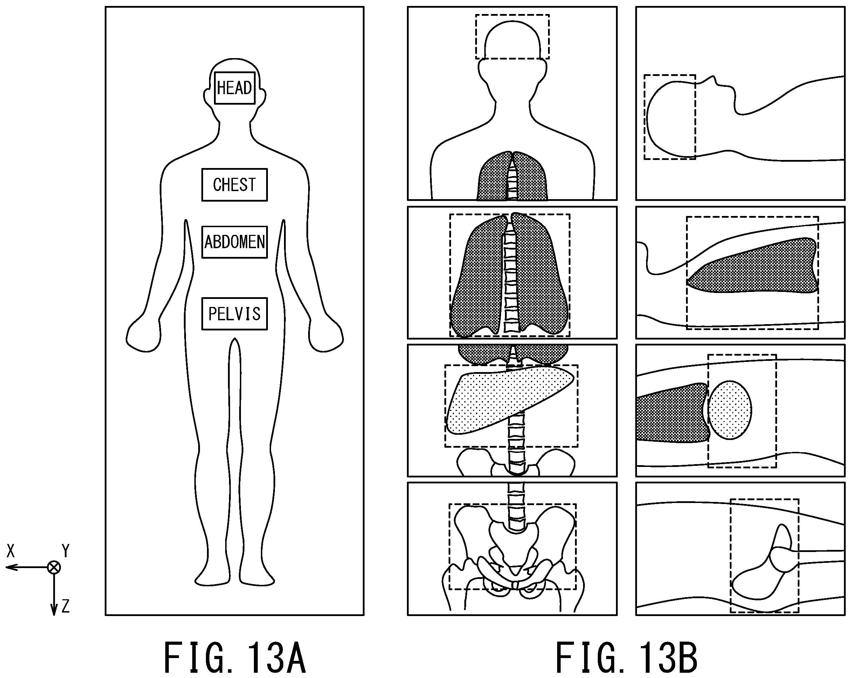

Fig. 13A shows an object with examination portions of the head, chest, abdomen, and pelvis placed on the top plate 14a. Fig. 13B shows the front and side virtual medical images (within the solid line frame) for each examination portion, which are generated based on the camera images for each examination portion in Fig. 13A, and the imaging range (within the dotted line frame) for acquiring the desired medical images. In such way, the imaging condition setting function F04 sets the second imaging condition for each examination portion based on the virtual medical image for each examination portion.

Since the amount of fat and muscle in the object differs depending on the examination portion, and the degree of X-ray penetration differs depending on the amount of fat and muscle, the X-ray dose irradiated during CT imaging may be changed depending on the examination portion. The imaging condition setting function F04 may set the second imaging condition for each examination portion such that the X-ray dose is appropriate for the examination portion.

The third acquiring function F07 acquires the imaging condition for each examination portion that has been input to the operation unit 33. The imaging condition setting function F04 resets the second imaging condition for each examination portion based on the imaging condition for each examination portion that has been input to the operation unit 33. The output function F06 controls the medical imaging apparatus 10 to output the second imaging condition for each examination portion. The output function F06 controls the position or angle of at least one of the X-ray irradiation unit 11 and the X-ray detector for each examination portion based on the second imaging condition. Specifically, the output function F06 controls the imaging range, imaging distance, position of the tube, and angle of the tube for each examination portion.

### (Second embodiment)

In some cases, it is desirable to acquire images of an object in a specified posture during an examination using the medical imaging apparatus 10. In such cases, there are objects that can be in the specified posture and objects that cannot be in the specified posture due to differences in the posture constraint conditions between the objects. The second embodiment differs from the first embodiment in that a second imaging condition is set based on the object information in addition to the virtual medical image.

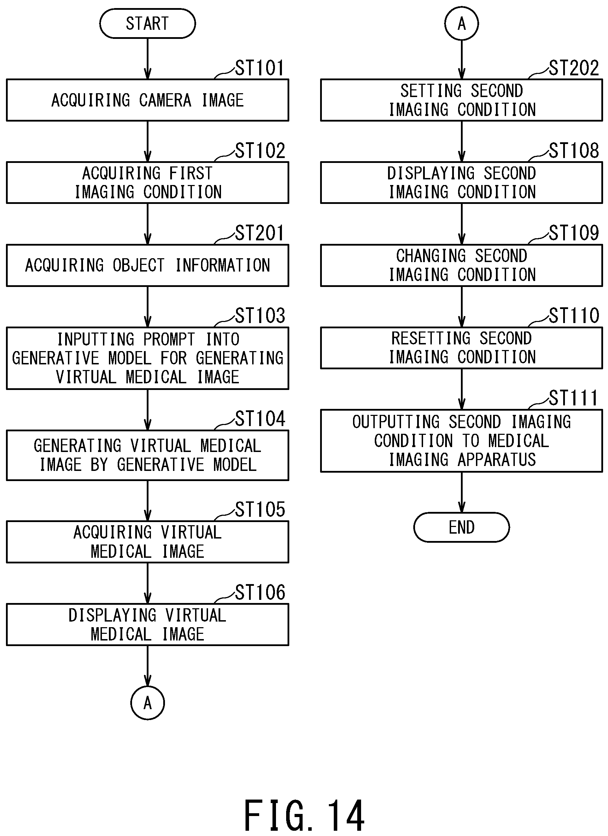

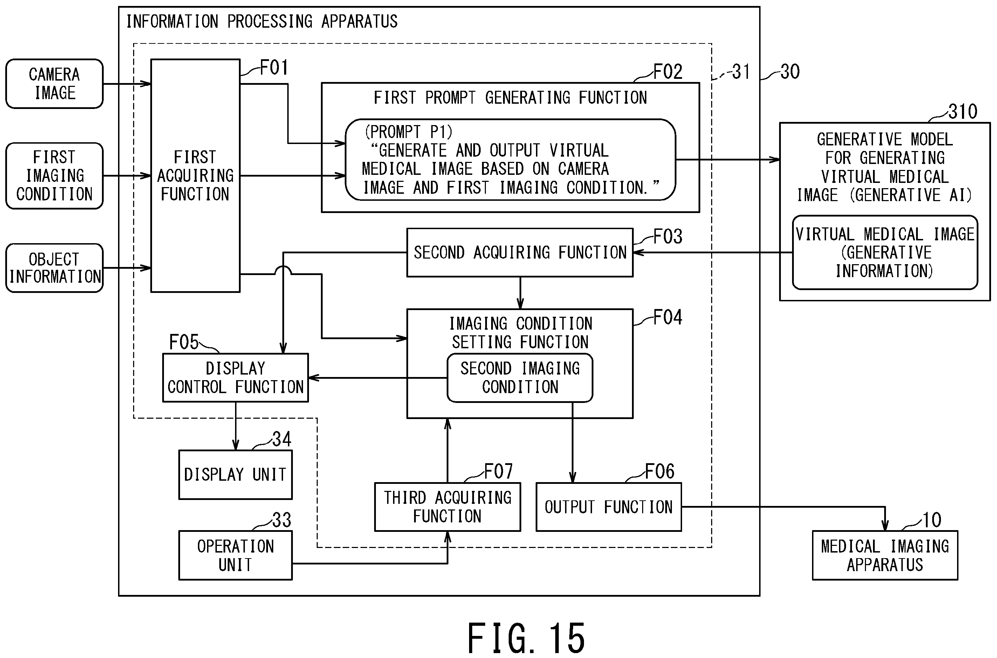

The object information includes information about the posture of the object. Information about the posture of the object includes, for example, constraints on the posture of the object and the current posture of the object. The object information may also include information about the object, such as the object's age, gender, height, weight, body fat percentage, and medical history. Referring to the flowchart in Fig. 14 and the explanatory diagram in Fig. 15, the functions and operations of the information processing apparatus 30 or the program related to the second embodiment will be described below. In the second embodiment, step ST202 is executed to set the second imaging condition, and step ST201 is executed at least before step ST202.

In step ST201, the first acquiring function F01 acquires object information. The first acquiring function F01 acquires object information from a hospital information system (HIS) or a radiology information system (RIS) connected via a network, for example.

At step ST202, the imaging condition setting function F04 sets the second imaging condition based on the object information and the virtual medical image. When the first imaging condition is determined using the virtual medical image to be appropriate for acquiring the desired medical image, the first imaging condition is set as the second imaging condition as is.

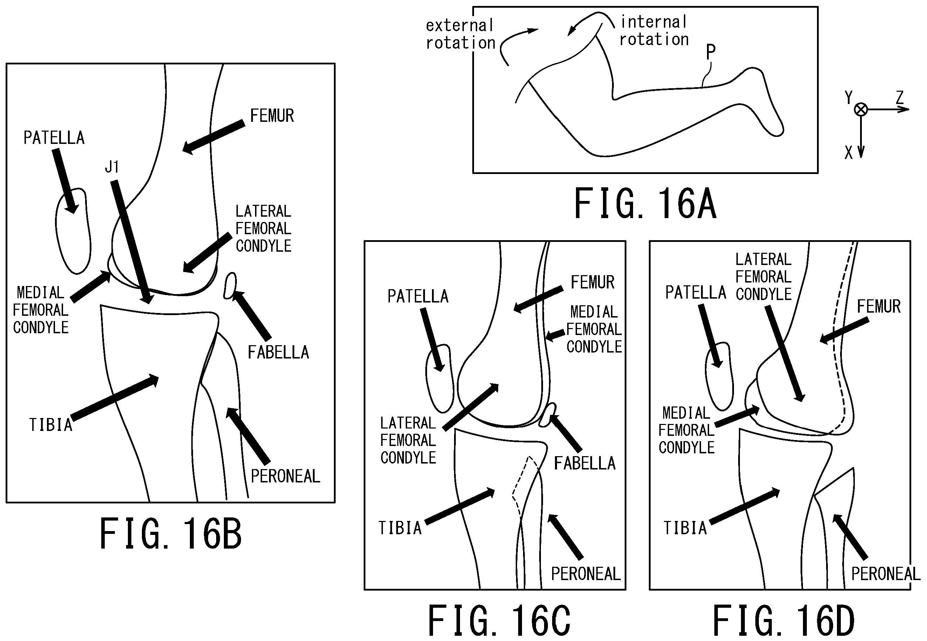

Fig. 16A shows a lateral imaging of a right knee with a flexion. Fig. 16B shows a virtual medical image of a lateral view of the knee joint imaged in the desired posture for the examination. In Fig. 16B, the lateral femoral condyle and medial femoral condyle overlap appropriately, the articular fissure J1 is located near the center of the virtual medical image, and the proximal ends of the tibia and peroneal overlap appropriately.

In contrast, when the knee is excessively internally rotated, the virtual medical image will look like the one shown in Fig. 16C. Specifically, the medial femoral condyle and lateral femoral condyle do not overlap but are offset, and the medial femoral condyle and lateral femoral condyle overlap the fabella, and the proximal end of the tibia and peroneal overlap excessively. The medial femoral condyle is offset to the right of the lateral femoral condyle in Fig. 16C.

When the knee is excessively externally rotated, the virtual medical image will be as shown in Fig. 16D. Specifically, the medial femoral condyle and lateral femoral condyle do not overlap but are offset (in Fig. 16D, the medial femoral condyle is offset to the left of the lateral femoral condyle), and the tibia and proximal end of the peroneal will not overlap and will be separated.

In cases where the knee is excessively internally or externally rotated, it is clear from the virtual medical image that the desired medical image cannot be obtained in the current posture, and it is necessary to change the posture of the object, the second imaging condition, or both. For example, when the knee is excessively internally rotated, the knee of the object may be externally rotated, or the angle of the tube may be changed.

When the object can externally rotate or internally rotate its legs and bend its knees, it is possible to have the object adopt an appropriate posture according to the content of the examination. However, there may be cases where the object cannot externally rotate or internally rotate its legs or bend its knees due to its own specific circumstances, such as pain when the knee is bent more than 90 degrees, and in such cases, it is difficult to have the object adopt the appropriate posture. Therefore, the information processing apparatus 30 of the second embodiment sets the second imaging condition while satisfying the constraint conditions of the posture of the object.

For example, in the case of imaging of a knee joint, when the object cannot change its posture, the imaging condition setting function F04 sets a second imaging condition that enables acquisition of the desired medical image without changing the posture. When the object can change its posture to some extent but has posture constraints, the imaging condition setting function F04 sets a second imaging condition that enables acquisition of the desired medical image by changing the posture within the range that satisfies the posture constraints, or without changing the posture.

When the object does not have any posture constraints and its posture can be changed in any way, the imaging condition setting function F04 can set a second imaging condition that allows the desired medical image to be acquired by changing the posture. In this case, a second imaging condition that allows the desired medical image to be acquired without changing the posture can also be set. In addition, the display control function F05 can give specific instructions for adjusting the posture via the display unit 34. In other words, it is possible to set the second imaging condition and give instructions via display unit 34 for adjusting the posture based on the object information about the posture of the object.

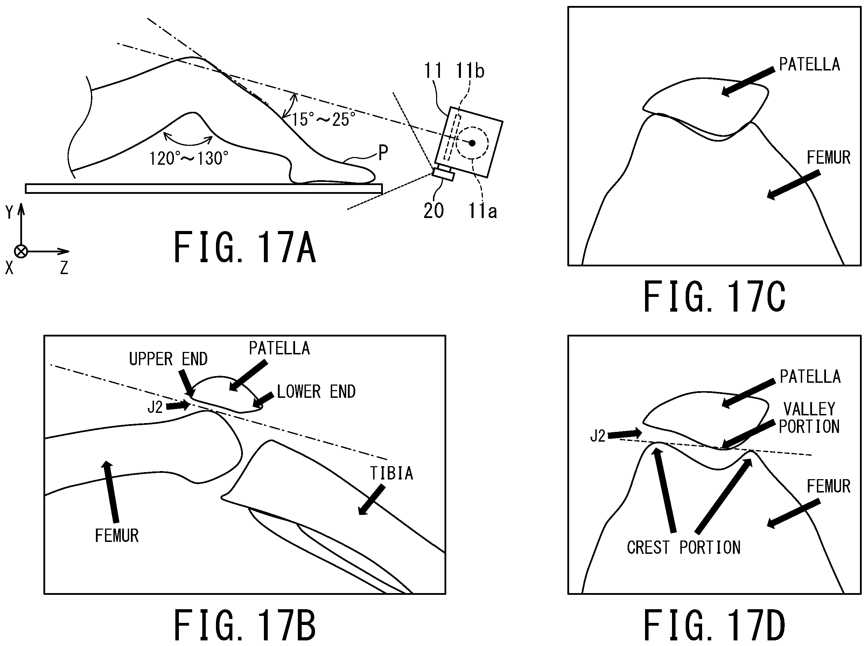

Fig. 17A to Fig. 17D explain the settings for the second imaging condition using specific examples. In frontal knee joint imaging, the knee joint axis position imaging (i.e. skyline method) is sometimes used. In the skyline method, by adjusting the degree of knee bending and the angle of X-ray incidence, it is desirable to acquire medical images in which the crest portion of the tibia and the valley portion of the femur are of similar height, the upper and lower edges of the patella do not overlap, and the gap J2 between the femur and patella is visible. Fig. 17A shows an example of an X-ray being incident at an angle of about 15 to 25 degrees from the tibial surface, with the knee bent such that the femur and tibia are at an angle of about 120 to 130 degrees.

Fig. 17B is a virtual medical image of a lateral imaging of the knee joint generated based on the camera image in Fig. 17A. The dotted line in Fig. 17B indicates the desirable angle of incidence of X-rays using the skyline method. Figs. 17C and 17D show virtual medical images of a frontal imaging of the knee joint generated based on the camera image in Fig. 17A. In the case of frontal radiography of the knee joint, it is desirable that the medical image is acquired such that the femur and patella do not overlap, i.e., such that the gap J2 between the femur and patella is legible. In the case of lateral radiography of the knee joint, the medical image is preferred to be acquired such that the lateral femoral condyle and medial femoral condyle overlap to a moderate extent and the articular fissure J1 is legible.

Fig. 17C is not set to the angle of the tube that is considered to be desirable in the skyline method. Specifically, because the femur and patella overlap, the imaging conditions for frontal knee joint imaging are appropriate, but the angle of the tube is not appropriate. In this case, the imaging condition setting function F04 calculates the angle of the tube such that the overlap between the femur and patella in the virtual medical image is eliminated, based on the state of overlap between the femur and patella.

For example, when the object can only bend its knee joint up to 150 degrees as a posture constraint, the imaging condition setting function F04 calculates the angle of the tube such that the overlap between the femur and patella is eliminated while the object is bent up to 150 degrees. Similarly, when the object can only bend the knee joint to between 120 and 130 degrees, the imaging condition setting function F04 calculates the angle of the tube such that the overlap between the femur and patella is eliminated when the object bent the knee joint to between 120 and 130 degrees. The angle of the tube can be calculated such that the overlap between the femur and patella is eliminated regardless of the angle the object can bend the knee joint to. In the case where femur and patella do not overlap in the virtual medical image, the imaging condition setting function F04 determines the angle of the tube as it is without change as the second imaging condition.

Fig. 17D shows the angle of the tube set to the angle considered to be desirable using the skyline method. Specifically, the angle of the tube is set, for example, by rotating 20 degrees around the X-axis, so that the overlap between the femur and patella is eliminated. In Fig. 17D, the overlap between the femur and patella is eliminated, and the gap J2 between the femur and patella is made readable.

The angle of the tube can be set based on the virtual medical images generated by rotating the angle of the tube by a specified value. The imaging condition setting function F04 sets the angle of the tube of the virtual medical image that is closest to the desired medical image as the second imaging condition.

For example, among the multiple virtual medical images generated by rotating the angle of the tube from 0 degrees clockwise around the X-axis in increment of 5 degrees, the angle of the tube of the virtual medical image in which the gap J2 between the femur and patella can be read is selected and set as the second imaging condition. In this case, the angle of the tube of the virtual medical image in which the gap J2 between the femur and patella is widest may be set, or an interpolated value between angles may be set, rather than a predetermined value for generating virtual medical images for each angle.

According to the second embodiment, the second imaging condition is set within the range of the posture of the object constraint conditions, and thus, desired medical images can be acquired not only for objects that can be arranged in a predetermined posture, but also for objects that cannot be arranged in a predetermined posture.

### (Variation of the second embodiment)

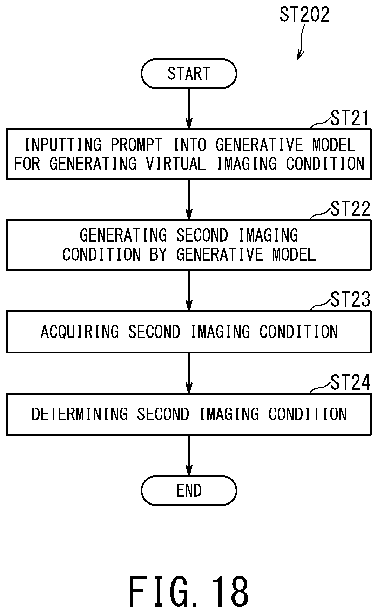

The variant of the second embodiment differs from the second embodiment in that the second imaging condition is calculated by the generative model 320 for generating imaging condition. Referring to the flowchart of Fig. 18 and the explanatory diagram of Fig. 19, the functions and operations of the information processing apparatus 30 or the program related to the variant of the second embodiment will be explained. The flowchart of Fig. 18 realizes the step ST202 of the flowchart of Fig. 14.

In step ST21, the second prompt generating function F08 inputs the prompt P2, which includes object information and a virtual medical image, into the generative model 320 for generating imaging condition. In this case, for example, the prompt P2 that gives the instruction "Please generate and output the second imaging condition based on the virtual medical image and object information" is executed.

At step ST22, the generative model 320 for generating imaging condition generates the second imaging condition according to the prompt P2. For example, the network interface 35 exchanges information and data with the generative model 320 for generating imaging condition. The generative model 320 for generating imaging condition may be partially or fully provided in the processing circuitry 31 or the memory 32.

The generative model of the imaging condition 320 is a generative model constructed using existing medical images and the imaging condition as training data, and it generates the second imaging condition based on the prompt P2. The generative model 320 for generating imaging condition may be a generator of a generative adversarial network, or a trained model learned using a variational autoencoder, or a pre-trained model learned using a diffusion model.

In step ST23, the fourth acquiring function F09 acquires the second imaging condition, which is the generated information generated by the generative model 320 for generating imaging condition.

In step ST24, the imaging condition setting function F04 determines and sets the second imaging condition. According to the variant of the second embodiment, since the second imaging condition is generated by the generative model 320 for generating imaging condition, the second imaging condition can be set more easily, regardless of the experience of the user.

According to at least one of the embodiments described above, the imaging condition can be set to acquire the desired medical image without increasing the radiation exposure.

The processing circuitry in the above-described embodiments is an example of the processing circuitry described in the claims. In addition, the term "processor" used in the explanation in the above-described embodiments, for instance, refers to circuitry such as dedicated or general-purpose CPUs (Central Processing Units), dedicated or general-purpose GPUs (Graphics Processing Units), or ASICs (Application Specific Integrated Circuits), programmable logic devices including SPLDs (Simple Programmable Logic Devices), CPLDs (Complex Programmable Logic Devices), and FPGAs (Field Programmable Gate Arrays), and the like. The processor implements various types of functions by reading out and executing programs stored in the memory circuitry.

In addition, instead of storing programs in the memory circuitry, the programs may be directly incorporated into the circuitry of the processor. In this case, the processor implements each function by reading out and executing each program incorporated in its own circuitry. Moreover, although in the above-described embodiments an example is shown in which the processing circuitry configured of a single processor implements every function, the processing circuitry may be configured by combining plural processors independent of each other so that each processor implements each function of the processing circuitry by executing the corresponding program. When a plurality of processors is provided for the processing circuitry, the memory medium for storing programs may be individually provided for each processor, or one memory circuitry may collectively store programs corresponding to all the functions of the processors.

While certain embodiments have been described, these embodiments have been presented by way of example only, and are not intended to limit the scope of the invention. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. An information processing apparatus (30) comprising processing circuitry (31) configured to:
acquire a camera image including an examination portion of an object;
input a prompt including at least the camera image into a generative model to acquire a virtual medical image from the generative model;
set a main imaging condition, which is an imaging condition used for a main imaging, based on the virtual medical image; and
output the main imaging condition to a medical imaging apparatus.

2. The information processing apparatus (30) according to claim 1, wherein the virtual medical image is a medical image generated based on a provisional imaging condition.

3. The information processing apparatus (30) according to claim 1, wherein the virtual medical image is a medical image imaged at a position and angle of an X-ray tube (11a) from which the camera image was acquired.

4. The information processing apparatus (30) according to any one of claims 1 to 3, wherein the processing circuitry (31) is configured to set the main imaging condition based on a geometric positional relationship between an imaging portion of the object and an X-ray tube (11a) used in the generation of the virtual medical image.

5. The information processing apparatus (30) according to any one of claims 1 to 4, wherein the processing circuitry (31) is configured to:
acquire a provisional imaging condition, and the camera image including the examination portion of the object;
input the prompt including the provisional imaging condition and the camera image to the generative model; and
acquire the virtual medical image from the generative model.

6. The information processing apparatus (30) according to any one of claims 1 to 5, wherein the imaging condition includes at least one of the following conditions: imaging range, imaging distance, position of an X-ray tube (11a), and angle of the X-ray tube (11a).

7. The information processing apparatus (30) according to claim 5 or 6, wherein the processing circuitry (31) is configured to acquire an object information including information about a posture of the object, and to set the main imaging condition based on the object information and the virtual medical image.

8. The information processing apparatus (30) according to claim 7, wherein the processing circuitry (31) is configured to input the prompt including the object information and the virtual medical image to the generative model for imaging conditions, and to set an imaging condition generated by the generative model of imaging conditions as the main imaging conditions.

9. The information processing apparatus (30) according to any one of claims 1 to 8, wherein the processing circuitry (31) is configured to calculate at least one of an imaging range, an imaging distance, a position of an X-ray tube (11a), and an angle of the X-ray tube (11a) to acquire a medical image based on a positional relationship between skeletal structure in the virtual medical image, and to set the calculated condition as the main imaging condition.

10. The information processing apparatus (30) according to any one of claims 1 to 9, wherein the processing circuitry (31) is configured to set the main imaging condition by:
acquiring the camera image for one examination portion of the object and input the prompt including the camera image to the generative model to acquire a plurality of virtual images from the generative model; and
selecting one imaging condition for acquiring a desired medical image from a plurality of imaging conditions for the respective plurality of virtual medical images.

11. The information processing apparatus (30) according to any one of claims 1 to 9, wherein the processing circuitry (31) is configured to:
acquire a plurality of camera images, wherein there is a camera image for each examination portion for a plurality of examination portions of the object;
input the prompt including the plurality of camera images to the generative model to acquire the virtual medical image for each examination portion from the generative model;
set the main imaging condition for each examination portion based on the virtual medical image for each examination portion; and
output the medical imaging condition for each examination portion.

12. A medical imaging system, comprising:
the information processing apparatus (30) according to any one of claims 1 to 11;
a medical imaging apparatus (10) connected to the information processing apparatus via a network; and
an image sensor (20) connected to the information processing apparatus via the network.

13. An image processing method, comprising:
acquiring a camera image including an examination portion of an object;
inputting a prompt including at least the camera image into a generative model to acquire a virtual medical image from the generative model;
setting a main imaging condition, which is an imaging condition used for a main imaging, based on the virtual medical image; and
outputting the main imaging condition to a medical imaging apparatus.

14. An image processing program for causing a computer to perform the following steps:
acquire a camera image including an examination portion of an object;
input a prompt including at least the camera image into a generative model to acquire a virtual medical image from the generative model;
set a main imaging condition, which is an imaging condition used for a main imaging, based on the virtual medical image; and
output the main imaging condition to a medical imaging apparatus.

15. An X-ray imaging system comprising X-ray irradiator (11) irradiates X-rays to an object, X-ray detector (13a, 15a) detects the X-rays transmitted through the object, and processing circuitry configured to:
acquire a camera image including an examination portion of the object;
input a prompt including at least the camera image into a generative model to acquire a virtual medical image from the generative model;
set a main imaging condition, which is an imaging condition used for a main imaging, based on the virtual medical image; and
control a position or angle of at least one of the X-ray irradiator (11) and the X-ray detector (13a, 15a) based on the main imaging condition.
